# EUROPEAN PATENT APPLICATION

(11) **EP 4 286 991 A1**
(43) Date of publication of application: **06.12.2023**
(21) Application number: 22197394.4
(22) Date of filing: 23.09.2022
(51) Int. Cl.: G06F 3/01, A61B 6/00, G06F 3/04815

(54) **GUIDANCE FOR MEDICAL INTERVENTIONS**

(30) Priority: 01.06.2022 US 202263347703 P
(71) Applicant: Koninklijke Philips N.V., 5656 AG Eindhoven (NL)
(72) Inventor: MORY, Benoit, Eindhoven (NL); WEBB, Penelope Eugenia, Eindhoven (NL); SALEHI, Leili, 5656AG Eindhoven (NL); KEENAN, Philip Joseph Jr., 5656AG Eindhoven (NL); PANSE, Ashish Sattyavrat, Eindhoven (NL)
(74) Representative: Philips Intellectual Property & Standards

(57) **Abstract**

The present invention relates to imaging during medical interventions. In order to provide facilitated guidance for an improved user comfort, a device (10) for guidance in medical interventions is provided that comprises a data input (12), a data processor (14) and an output interface (16). The data input is configured: to provide 3D image data of a region of interest of a subject; to provide a zoom factor and a bounding box around the determined location; and to receive a user interaction for defining locations within the region of interest in the 3D image data. The data processor is configured: to determine, by the user interaction with a presented first view, a location within the presentation of the region of interest as zoom center point for a zoomed second view; to zoom-in of the 3D image data according to the zoom factor; to crop-off content of the 3D image data outside the bounding box defining a new region of interest inside the bounding box; and to generate the zoomed second view for the new region of interest based on the zoom factor and the 3D image data within the bounding box. The output interface is configured: to provide, based on the 3D image data, presentation data for the region of interest in the first view; and to provide presentation data for the new region of interest in the zoomed second view.

## Description

### FIELD OF THE INVENTION

The present invention relates to imaging during medical interventions and relates in particular to a device for guidance in medical interventions, to a system for guidance in medical interventions and to a method for guidance in medical interventions.

### BACKGROUND OF THE INVENTION

Viewing 3D objects in stereoscopic space allows observation of an entire model at a chosen size and distance from user's point-of-view. However, to see specific areas in more detail, e.g. a vascular structure, the user would either need to step closer to the model or bring the model closer to them. Depending on the context of the space or situation in which they are using the application, this may be impractical or not possible.

Zooming/scaling is a feature on existing 2D user interfaces. For instance, some applications allow to zoom into an area by pointing the mouse and using the scroll wheel, or on a phone or tablet, double tap on an area and drag finger up/down. Other applications allow selecting an area, then clicking and holding the mouse, moving it up/down to scale the page up/down. Further applications, when viewing images or documents, allow zooming by making a two-finger pinching action. Other systems allow increasing the size of the screen content using the keyboard keys 'command +', or 'ctrl +'. In 3D, in some examples, zooming on the surface of a 3D model is achieved by pressing a key and dragging using the left and middle mouse buttons together. As another example, WO 2019/043025 A1 describes zooming an omnidirectional image.

### SUMMARY OF THE INVENTION

However, there is a need to provide facilitated guidance for an improved user comfort.

The object of the present invention is solved by the subject-matter of the independent claims; further embodiments are incorporated in the dependent claims. It should be noted that the following described aspects of the invention apply also for the device for guidance in medical interventions, for the system for guidance in medical interventions and for the method for guidance in medical interventions.

According to the present invention, a device for guidance in medical interventions is provided. The device comprises a data input, a data processor and an output interface. The data input is configured to provide 3D image data of a region of interest of a subject. The data input is also configured to provide a zoom factor and a bounding box around the determined location. The data input is further configured to receive a user interaction for defining locations within the region of interest in the 3D image data. The data processor is configured to determine, by the user interaction with a presented first view, a location within the presentation of the region of interest as zoom center point for a zoomed second view. The data processor is also configured to zoom-in the 3D image data according to the zoom factor. The data processor is further configured to crop-off content of the 3D image data outside the bounding box defining a new region of interest inside the bounding box and to generate the zoomed second view for the new region of interest based on the zoom factor and the 3D image data within the bounding box. The output interface is configured to provide, based on the 3D image data, presentation data for the region of interest in the first view. The output interface is also configured to provide presentation data for the new region of interest in the zoomed second view.

As an effect, providing intuitive interaction techniques for targeted zooming into anatomical details in the context of interventional procedures improves the usability of volumetric 3D models in mixed reality. In an example, 3D holographic content is magnified and cropped by pointing at a 3D position, e.g. finger pointing or eye gaze, and triggering a zoom operation, e.g. with a voice command. The scaled volume will automatically be positioned in the center of the virtual bounding box based on the location pointed by user.

According to an example, the output interface is configured to present the first view to the user in a 3D mixed reality presentation. The data input is configured to receive user interaction from at least one of the group of: manually pointing to a location within the 3D mixed reality presentation and pointing tracking, eye gazing for identifying the location within the 3D mixed reality presentation, and voice commands.

According to an example, the output interface is configured to present the 3D mixed reality presentation as a presentation of the 3D image data as an overlay to reality such that the user can observe his/her hands within the presentation of the 3D image data. The data input is configured to receive 3D hand tracking data from a measurement of a spatial position of the user's hand in relation to the presentation of the 3D image data.

According to an example, the output interface is configured to provide the first view comprising segmented anatomical structures. The output interface is configured to provide the first view as 3D presentation to the user. The data input is configured to receive the user interaction as spatial related user interaction provided within the 3D presentation.

According to an example, the data input is configured to provide anatomical image content data for a predefined region around the determined location. The data processor is configured to identify anatomical structures based on the determined location and the anatomical image content data. The data processor is configured to adjust, based on the identified anatomical structures, at least one of the group of location, bounding box and zoom center point. The data processor is also configured to generate at least one of the group of an adjusted zoom center point and an adjusted bounding box such that a defined part of the anatomical structures is less cropped in the second view.

According to an example, the data processor is configured to identify an anatomical portion. The data processor is also configured to at least one of the group of the following: adjust the zoom center point to a center of the anatomical portion and adjust the bounding box and/or the zoom factor to enclose a maximum of the anatomical portion.

According to an example, the data processor comprises a convolutional neural network configured to perform, based on the 3D image data, at least one of the group of the following: annotate the 3D image data, determine a zoom factor, identify a procedure type, zoom-in and crop-off of content outside the bounding box.

According to an example, the data input is configured to provide markers as labels of regions of interest of the 3D image data. The markers are provided as identifiable zoom center points for the second view.

According to the present invention, a system for guidance in medical interventions is also provided. The system comprises a display arrangement, a user input device and a device for guidance in medical interventions according to one of the preceding examples. The display is data-connected to the device for guidance in medical interventions and is configured to provide a 3D presentation of a region of interest of a subject. The user input device is data-connected to the device for guidance in medical interventions and is configured for user interaction to determine a location within the 3D presentation.

According to an example, the display arrangement comprises a stereo display configured to present the 3D image data for spatial user action within the presented 3D image.

According to an example, the display arrangement is a mixed reality display configured to present the 3D image data overlaid to reality for user interaction by 3D pointing at a location within the 3D image. The user input device comprises a detector for detecting the pointing of the user.

According to the present invention, a method for guidance in medical interventions is also provided. The method comprises the following steps:
- providing 3D image data of a region of interest of a subject;
- presenting, based on the 3D image data, the region of interest in a first view;
- determining, by user interaction with the presented first view, a location within the presentation of the region of interest as zoom center point for a zoomed second view;
- providing a zoom factor and a bounding box around the determined location;
- zooming-in of the 3D image data according to the zoom factor;
- cropping-off content of the 3D image data outside the bounding box defining a new region of interest inside the bounding box;
- generating the zoomed second view for the new region of interest based on the zoom factor and the 3D image data within the bounding box; and
- providing the zoomed second view to the user.

An example for a field of use is mixed reality for viewing and interacting with 3D volumes for image guided therapy.

According to an aspect, *zooming,* as provided in 3D by the present invention, refers to an operation that not only rescales but also crops out, or crops-off, any content that falls outside a predefined initial frame (e.g. a 3D bounding box that plays the same role as a 2D window on a screen). Contrary to this, *scaling* magnifies the entire content uniformly. The zooming, however, keeps a frame around the content fixed and crops out anything that falls outside that frame after magnification.

In an example, also an interventional imaging system is provided that generates the 3D images, which images can be manipulated using the system described above. Examples of such systems are: fixed monoplane and biplane C-arm X-ray systems, mobile C-arm X-ray systems, CT scanners, MRI scanners or 3D ultrasound scanners.

An example of application is viewing and assessment of preoperative and intraoperative 3D volumetric datasets for image guided therapy (IGT). The volumetric datasets are intended to be displayed during an interventional procedure inside the cathlab, the user wears the mixed reality headset, where they can view and interact with the procedures described above prior to, or during a procedure. The use of the zoom functionality is specific to the assessment of anatomical datasets, to be able to inspect a specific anatomical region in greater detail, or for the purpose of communication of fellow staff.

In an example, the use requires an interactive, stereoscopic display, and is therefore applicable to any 3D volume AR/VR (augmented reality/virtual reality) viewing application that utilizes an interactive holographic display.

With this invention aimed at stereoscopic 3D applications, it is possible to place the center of the zoom anywhere in 3D space, even inside objects.

In an aspect, a 3D scaling operation in mixed reality is provided. For instance, the system can zoom into a region of interest in 3D and automatically bring it to the center of the field of view and crop the objects in the foreground and background of the target object.

As an effect, scaling up a 3D model in AR/VR is provided that allows to look at small details. By spatial cropping, any obscuring of the field-of-view by foreground features like peripheral vessels is avoided. For large magnification factors needed to view small anatomical details and pathologies, the cropping also eliminates the use of enlarged virtual models that take so much 3D space that they can cause discomfort. This is achieved by defining a small region-of-interest around the target structure, and/or by keeping the initial 3D bounding box size as a constant, comfortable value.

Instead of a complex interaction involving multiple steps, a single, fast interaction way is provided to simultaneously scale up to any magnification factor, re-center, and crop peripheral content in mixed reality. When zooming in 3D, magnification occurs in all directions. The cropping results in that it is avoided that the foreground parts of the 3D content move closer to the user, occluding the background and causing potential discomfort as some visible areas may be too close and fall outside of the optimal focal zone of the headset.

In an aspect, a cuboid box is maintained around the volume as constant, and only content inside that box is rescaled. Any 3D content falling outside the box after scaling will be cropped, ensuring that the physical space of the 3D virtual content in the room remains the same.

An effective zoom & crop mechanism in 3D is provided that assists and adapts to the content, in order to maximize visibility of potentially important structures and keep them in the 3D region of interest.

As an effect, for example for the use in mixed reality in a medical interventional environment, the space taken by virtual content is minimized to avoid obscuring the user's field-of-view unnecessarily, while still achieving large magnification factors on 3D volumes to display anatomical structures of interest with as many fine details as possible.

This approach allows users to view 3D volumes or volumetric regions of interest in stereoscopic space and gain better anatomical understanding for their procedures while working in a cathlab, for example. The ability to zoom into a specific volumetric region of interest provides users with beneficial means to quickly declutter 3D imagery, and quickly/intuitively focus on their volumetric region of interest using a simple target selection mechanism, e.g. based on eye-gaze or gestures and voice commands.

In an additional option, a context-adaptive approach is provided, in which the scaling factor and center can be automatically selected. In an example, this is achieved with statistical image analysis techniques or with more advanced machine learning techniques. For instance, a neural network is trained to create attention maps based on anatomical landmarks in various procedures. The attention maps display the most important area found by the network using information related to the procedure such as the area or organ where the procedure is performed on (e.g. brain, spine, heart, kidney, etc.), pre-operative notes or diagnoses (e.g. aneurysm, thrombus, occlusion, stone, etc.), current device locations (e.g. catheter tip, stent, coil, etc.), common practices and workflows (e.g. measuring the parent vessel diameter before aneurysm treatment) and the like.

The benefits of using an interaction method for zooming in 3D space over a peripheral tool on a 2D screen includes sterility, speed, usability, ease of understanding and quicker access to important clinical details.

These and other aspects of the present invention will become apparent from and be elucidated with reference to the embodiments described hereinafter.

### BRIEF DESCRIPTION OF THE DRAWINGS

Exemplary embodiments of the invention will be described in the following with reference to the following drawings:
Fig. 1 schematically shows an example of a device for guidance in medical interventions.
Fig. 2 shows an example of a system for guidance in medical interventions with a display arrangement provided as a mixed reality display.
Fig. 3 shows basic steps of an example of a method for guidance in medical interventions.
Fig. 4a shows an example of a 2D image (left part) and a presentation of 3D image data (right part) in an initial presentation; Fig. 4b shows a scaled presentation; and Fig. 4c shows a zoomed-in and cropped-off presentation.
Fig. 5a shows an example of image presentation steps comprising zooming and cropping in a starting presentation; Fig. 5b shows a partly zoomed-in presentation; and Fig. 5c shows a zoomed-in and cropped-off presentation.
Fig. 6a shows another example of image presentation steps comprising magnification, cropping and re-centering around a 3D target in a first state; Fig. 6b shows a partly zoomed-in state; and Fig. 6c shows a final zoomed-in and cropped-off state.
Fig. 7a shows an example of a fixed-size, non-adaptive zoom-in and centering scheme in an initial state; Fig. 7b shows a state of identified zoom field; and Fig. 7c shows a final zoomed-in and cropped-off state.
Fig. 8a shows an example of a content-adaptive zoom-in and centering scheme in an initial state; Fig. 8b shows a state of identified zoom field; Fig. 8c shows a re-centered zoom field; and Fig. 8d shows a final state.
Fig. 9 shows an example of a marker placed in a presented volume in a region of interest.

### DETAILED DESCRIPTION OF EMBODIMENTS

Certain embodiments will now be described in greater details with reference to the accompanying drawings. In the following description, like drawing reference numerals are used for like elements, even in different drawings. The matters defined in the description, such as detailed construction and elements, are provided to assist in a comprehensive understanding of the exemplary embodiments. Also, well-known functions or constructions are not described in detail since they would obscure the embodiments with unnecessary detail. Moreover, expressions such as "at least one of', when preceding a list of elements, modify the entire list of elements and do not modify the individual elements of the list.

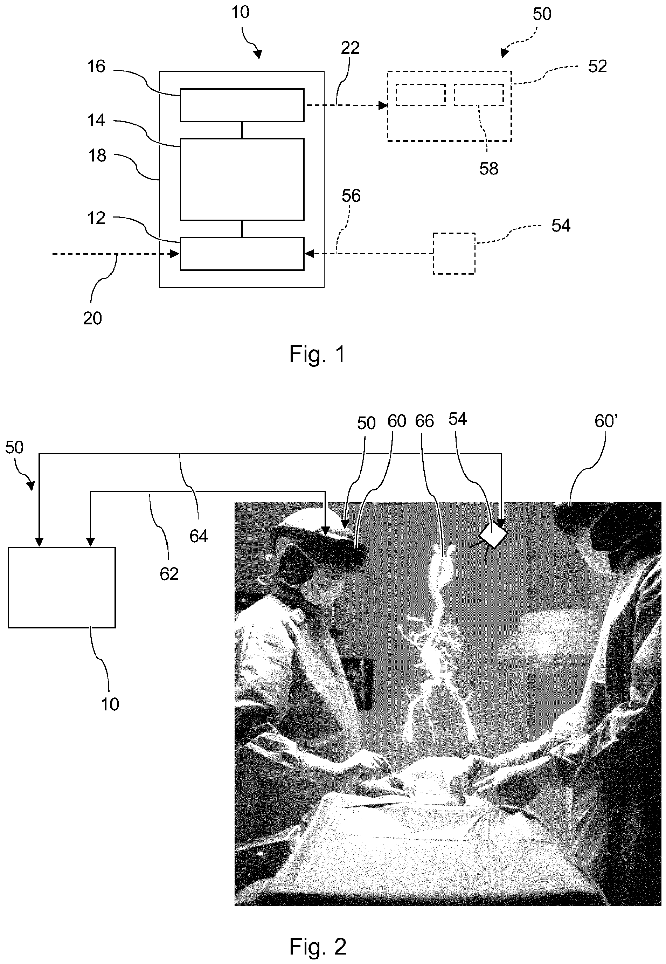

Fig. 1 schematically shows an example of a device 10 for guidance in medical interventions. The device 10 comprises a data input 12, a data processor 14 and an output interface 16. The data input 12 is configured to provide 3D image data of a region of interest of a subject. The data input 12 is also configured to provide a zoom factor and a bounding box around the determined location. The data input 12 is further configured to receive a user interaction for defining locations within the region of interest in the 3D image data. The data processor 14 is configured to determine, by the user interaction with a presented first view, a location within the presentation of the region of interest as zoom center point for a zoomed second view. The data processor 14 is also configured to zoom-in of the 3D image data according to the zoom factor. The data processor 14 is further configured to crop-off content of the 3D image data outside the bounding box defining a new region of interest inside the bounding box. The data processor 14 is furthermore configured to generate the zoomed second view for the new region of interest based on the zoom factor and the 3D image data within the bounding box. The output interface 16 is configured to provide, based on the 3D image data, presentation data for the region of interest in the first view. The output interface 16 is also configured to provide presentation data for the new region of interest in the zoomed second view.

The data input 12, the data processor 14 and the output interface 16 can be provided in a common structure, like a common housing, as indicated by a frame 18, or even in an integrated manner. In a further option (not shown), they are provided as separate components or units.

A first arrow 20 indicates data supply to the data input 12. A second arrow 22 indicates data supply from the output interface 16. The data supply can be provided wire-based or wireless.

The term "data input" relates to providing or supplying data for data processing steps. The data input can also be referred to as image data input. The data input can also be referred to as data supply, as image data supply, as image input, as input unit or simply as input. In an example, the image data input is data-connectable to an imaging source arrangement. In an example, the data input is data-connectable to a data storage having stored the image data.

The term "data processor" relates to a processor or part of a processor arrangement that is provided to conduct the computing steps using the data supplied by the data input. The data processor can also be referred to as data processing arrangement, as processor unit or as processor. In an example, the data processor is data-connected to the data input and the output interface.

The term "output interface" relates to an interface for providing the processed or computed data for further purposes. The output interface can also be referred to as output or output unit. In an example, the output interface is data-connectable to a display arrangement or display device. In another example, the output is data-connected to a display.

In an option, the zoom factor is pre-determined.

In another option, the bounding box is pre-determined.

The term "cropping-off' relates to deleting the image data outside the bounding box for the purpose of presenting the 3D image.

The content is cropped-off in particular in front of and on the back of the bounding box (front and back relating to the viewing direction).

As an option, Fig. 1 also shows (in hashed lines) an example of a system 50 for guidance in medical interventions with a display arrangement 52 provided as a mixed reality display. The system 50 also comprises a user input device 54 and the device 10 for guidance in medical interventions. The display arrangement 52 is data-connected to the device 10 for guidance in medical interventions, as indicated by the second arrow 22. The display arrangement 52 is configured to provide a 3D presentation of a region of interest of a subject. The user input device 54 is data-connected, as indicated by a third second arrow 56, to the device 10 for guidance in medical interventions and is configured for user interaction to determine a location within the 3D presentation.

The user input device 54 provides the data to the data input 12 of the device 10 for guidance in medical interventions. The display arrangement 52 receives the respective data from the output interface 16 of the device 10 for guidance in medical interventions.

In an option, the display arrangement 52 comprises a stereo display 58 configured to present the 3D image data for spatial user action within the presented 3D image.

Fig. 2 shows an example of the system 50 for guidance in medical interventions where the display arrangement 52 is a mixed reality display 60 configured to present the 3D image data overlaid to reality for user interaction by 3D pointing at a location within the 3D image. The user input device 54 is provided, for example, as a detector for detecting the pointing of the user. For example, the detector is provided as a camera for tracking the user's hand and finger movements. The mixed reality display 60 is data-connected to the device 10 for guidance in medical interventions, as indicated by a first line 62. The user input device 54 is data-connected to the device 10 for guidance in medical interventions, as indicated by a second line 64. The data-connection can be provided wire-based or wireless.

For example, the mixed reality display 60 is provided as a head-mounted viewing device. In one option, the mixed reality display 60 is a virtual reality display. The user's hand is shown within a virtual display of the anatomical structure. In another option, the mixed reality display 60 is an augmented reality display. The user's hand is visible as reality and the display of the anatomical structure is overlaid to provide augmented reality.

As indicated in Fig. 2, an anatomical structure 66 is presented to the user via the mixed reality display 60. In an example, the anatomical structure 66 is only visible to the user. The user can then point at a certain point of interest within the anatomical structure 66 for zooming-in and cropping-off of the anatomical structure 66 outside a determined bounding box.

In an option, the anatomical structure 66 is also visible to other users, for example via a further example 60' of the mixed reality display 60 worn by a second user.

In an option, the pointing is done manually, e.g. by the user's hand or fingers. The detector comprises a camera providing a stream of images of the user's hands and fingers or fingertips detection is provided for the stream of images. The detection comprises a spatial detection and a registration of the user's hand with the presented 3D image space is provided.

In another option (not shown in detail), the pointing is done by the tracking of the user's eyes and the respective viewing direction and focus within the 3D image.

In an example, a system is provided that comprises a stereoscopic rendering engine, a 3D pointing device and a zooming controller. The stereoscopic rendering engine is capable of displaying at any scale 3D datasets consisting of volumetric images, e.g., CT, MR, 3D ultrasound, or meshes that can be cropped to a 3D bounding box. The 3D pointing device is capable of providing user input as 3D coordinates in a referential that is co-registered with the content, e.g. a hand tracking software driver of an AR/VR headset. The zooming controller takes as input the 3D dataset and the 3D pointing device and outputs an estimate of the optimal shift and magnification factor to be applied to the content when the zoom-in operation is triggered. Further, a trigger event is provided to initiate the zoom-in operation, such as a voice command e.g. "zoom-in".

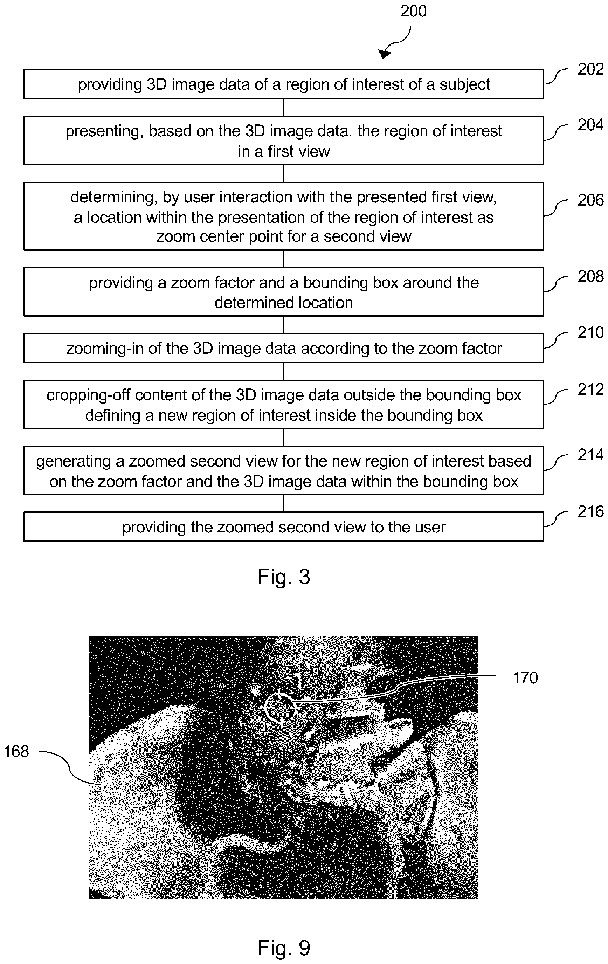

Fig. 3 shows basic steps of an example of a method 200 for guidance in medical interventions. The method 200 comprises the following steps: In a first step 202, 3D image data of a region of interest of a subject is provided. In a second step 204, based on the 3D image data, the region of interest is presented in a first view. In a third step 206, by user interaction with the presented first view, a location within the presentation of the region of interest is determined as zoom center point for a zoomed second view. In a fourth step 208, a zoom factor and a bounding box around the determined location are provided. In a fifth step 210, the 3D image data is zoomed-in according to the zoom factor. In a sixth step 212, content of the 3D image data is cropped-off outside the bounding box defining a new region of interest inside the bounding box. In a seventh step 214, the zoomed second view is generated for the new region of interest based on the zoom factor and the 3D image data within the bounding box. In an eighth step 216, the zoomed second view is provided to the user.

In an example of the method 200, the first view is presented to the user in a 3D mixed reality presentation. The user interaction comprises at least one of the group of: manually pointing to a location within the 3D mixed reality presentation and hand tracking; eye gazing for identifying the location within the 3D mixed reality presentation; and voice commands.

In an example of the method 200, the 3D mixed reality presentation comprises a mixed reality display to display a presentation of the 3D image data as an overlay to reality such that the user can observe his/her hands within the presentation of the 3D image data. Further, 3D hand tracking is provided to measure a spatial position of the user's hand in relation to the presentation of the 3D image data.

In an example of the method 200, at least one of the parameters of the group of pre-determined zoom factor and the pre-determined bounding box is adapted by the user by user interaction. The zoomed second view is generated based on the at least one adapted parameter.

In an example of the method 200, the first view comprises segmented anatomical structures. The first view is provided as 3D presentation to the user. Further, spatial related user interaction is provided within the 3D presentation.

In an example of the method 200, anatomical image content data is provided for a predefined region around the determined location. Based on the determined location and the anatomical image content data, anatomical structures are identified. Based on the identified anatomical structures, at least one of the group of location and bounding box is adjusted to generate at least one of the group of an adjusted zoom center point and an adjusted bounding box such that a defined part of the anatomical structures is less cropped less cropped in the zoomed second view.

In an example of the method 200, an anatomical portion is identified. The location is adjusted to a center of the anatomical portion. Alternatively or in addition, the bounding box is adjusted to enclose a maximum of the anatomical portion.

In an example, for activating the zooming-in and the cropping, a trigger event is provided that comprises at least one of the group of: voice command, eye gaze, gesture recognition and manual interface interaction.

In an example of the method 200, a convolutional neural network is provided for providing at least one of the following steps based on the 3D image data: annotating the 3D image data; determining a zoom factor; identifying a procedure type; zooming-in; and cropping-off of content outside the bounding box.

In an example of the method 200, regions of interest of the 3D image data are labelled with markers. The markers are provided as identifiable zoom center points for the second view.

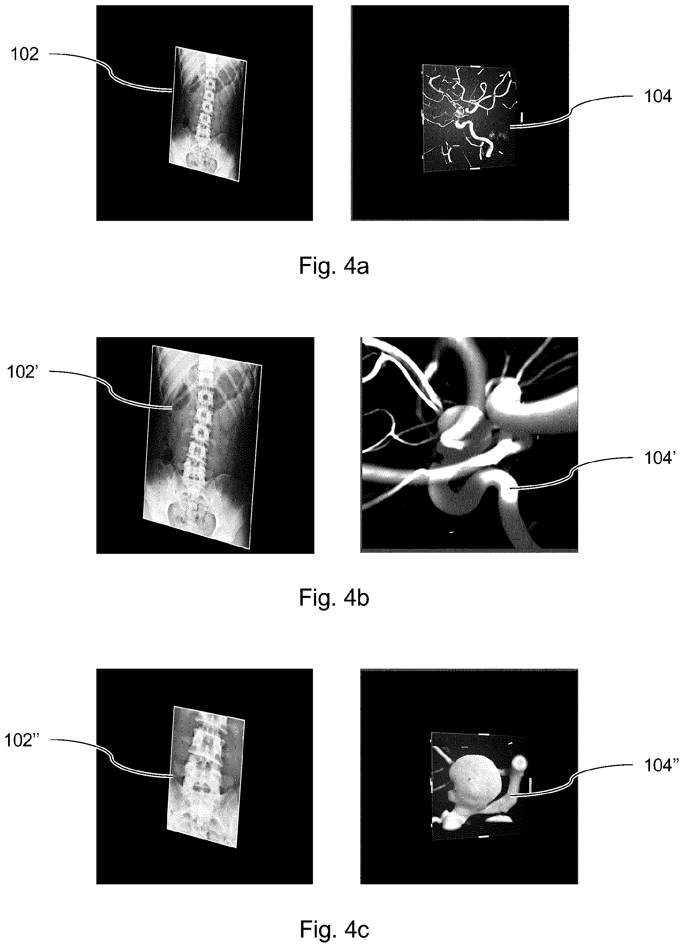

Fig. 4a shows, for comparison and explanation, an example of a 2D X-ray image 102 (left part) and a presentation of 3D image data 104 (right part) in an initial presentation. Fig. 4b shows a *scaled* presentation 102' of the 2D X-ray image and a *scaled* presentation 104' of the 3D image data. Fig. 4c shows a zoomed and cropped-off presentation 102" of the 2D X-ray image and a zoomed and cropped-off presentation 104" of the 3D image data provided as described above. As can be seen, in contrast to the scaled presentation of the 3D image data of Fig. 4b, the zoomed and cropped-off presentation 104" of the 3D image data enabled a much better view on the target, i.e. on the region of interest.

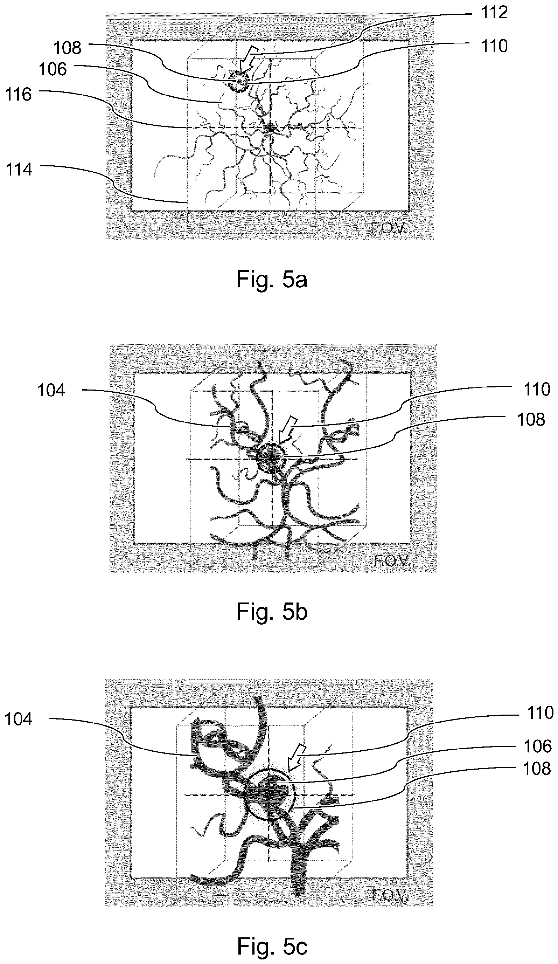

Fig. 5a shows an example of image presentation steps comprising zooming and cropping in a starting presentation. An anatomical structure 106 is shown in form of a vascular structure comprising an aneurysm 108, marked with a circle 110 and an arrow 112. A frame indicates a bounding box 114 of the spatial field of view. A cross marks a center 116 of the bounding box 114. Fig. 5b shows a partly zoomed-in presentation. The selected volumetric region of interest is moved to the center of the field of view. In the example shown, movement of the aneurysm 108 towards the center of a frame is provided. The frame is defined by the 3D bounding box 114 that remains fixed during the zoom operation. Fig. 5c shows a zoomed-in presentation with cropped-off structures outside the bounding box 114.

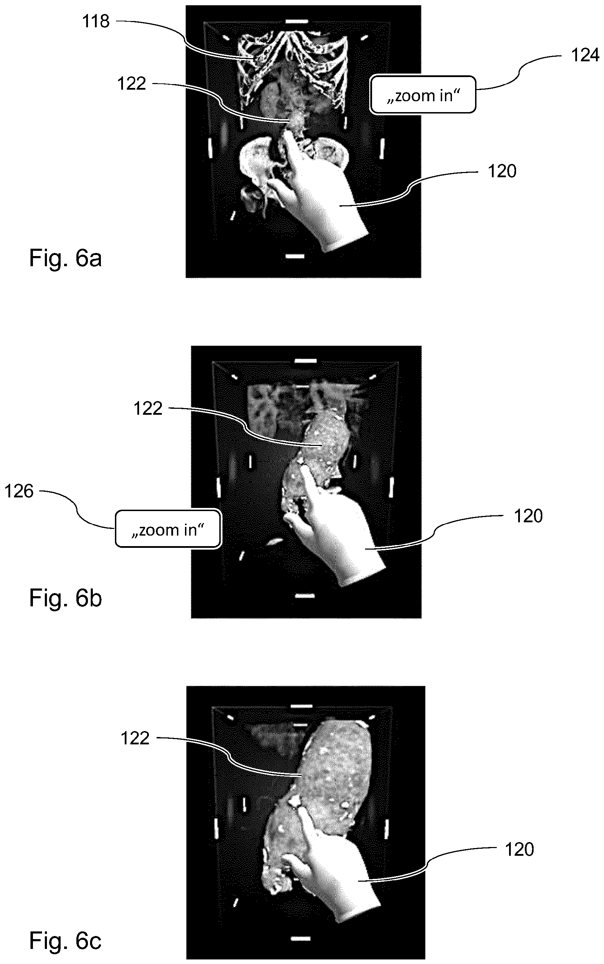

Fig. 6a to Fig. 6c show an example for a zooming-in procedure. Fig. 6a shows another example of an anatomical structure 118. A user's hand 120 is shown within the 3D presentation of the anatomical structure 118. The user can identify a part 122 of the anatomical structure 118 that is of interest for zooming-in. Upon identifying the part, the user can give a voice command 124, like "zoom-in", such that image presentation steps are provided comprising magnification, cropping and re-centering around the 3D target. In an option, magnification, cropping and re-centering around the 3D target defined by the user forefinger tip's 3D location and triggered by a voice command are provided simultaneously. Fig. 6b shows a partly zoomed-in state. The user can verify the location of interest by e.g. once again virtually touching the respective part of the presentation of the anatomical structure 118. Upon a further voice command 126, a further magnification, cropping and re-centering around the 3D target is provided. Fig. 6c shows the final state.

In an example, point & zoom is provided. A system is provided that utilizes the forefinger to point at a volumetric region of interest on a holographic model and that uses a voice command such as 'zoom-in' to scale the model up around the location of the fingertip. The zoom operation is targeted in such a way that the 3D region of interest that the user is pointing to will be centered, while the content around is scaled up. The bounding box of the volume remains fixed and the content expanding outside the box is cropped-off The advantage of this example is that both high levels of magnification and volumetric region of interest selection can be achieved without increasing the space that the hologram takes in the field of view. The interface for using this feature may consist only of the user's hand and their voice. The zooming feature can be used at any time within a volume viewer application, triggered by the detection of a pointing hand and the voice command, as shown in Fig. 6a to Fig. 6c.

The system continuously receives hand tracking information and voice input. When the user's hand is detected, each articulation of the hand is tracked by the head mounted device sensors and mapped to the virtual space of the 3D volume. When the trigger event is detected, e.g. 'zoom-in' voice command, the application scales, crops, and re-centers the volumetric region of interest that the user is targeting with their forefinger. Optionally, the user's real hand is highlighted by a virtual articulated mesh to give a better representation of their hand in the virtual space. Optionally, the zoom-in trigger launches a 3D continuous animation of a predefined duration, e.g. 1 sec, that interpolates from the current set of rendering parameters to the target zoom and center.

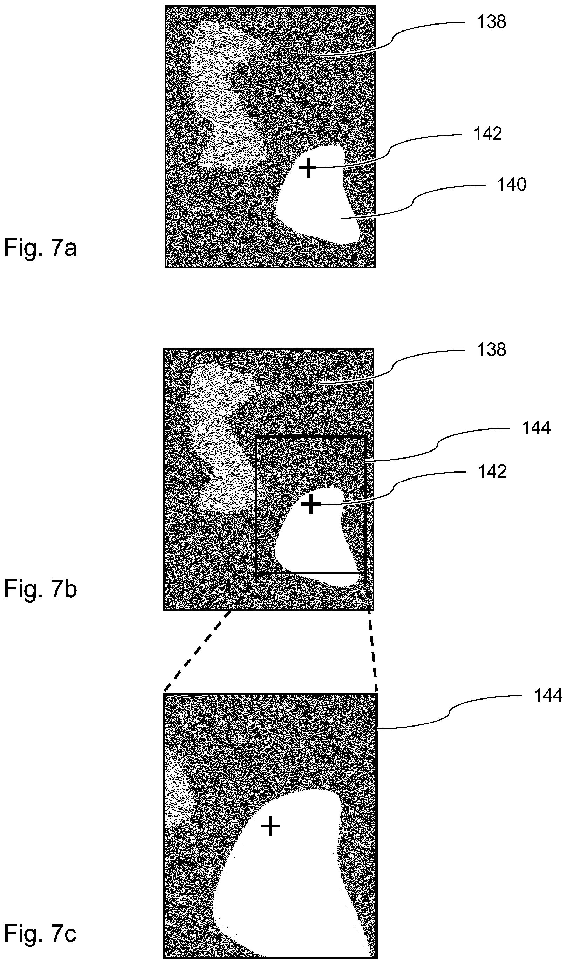

Fig. 7a to Fig. 7c show another example for a zooming-in procedure with a fixed-size, non-adaptive zoom-in and centering scheme. Fig. 7a shows an initial state with an anatomical structure 138 presented as 3D image with a portion of interest 140. A center mark 142 is arranged on the portion of interest 140. Fig. 7b indicates an identified (spatial) zoom field 144. Fig. 7c shows a final state where with cropped-off structures outside the zoom field 144. In the example shown in Fig. 7a to Fig. 7c, the zooming controller selects as center the location of the user fingertip. Content is magnified by a fixed, predefined factor, e.g. 2x, cropped and shifted so that the region of interest frame remains fixed in space and the target is at the center of the new region of interest. This works well in some cases but might have the unintended effect or cropping out some important structures on the sides of the bounding box.

In another example, eye gaze to center on the region of interest is provided. An alternative example is the use of eye gaze as a 3D pointing device to center on the region of interest. Using eye gaze allows the user to perform a hands-free interaction using just their eyes and a voice to manipulate the volume to the position they require. Changing the opacity of the objects using a window or a semi-transparent view allows for concentrating the eye gaze and hence magnifying and centering around the regions inside the objects rather than just the surface of the object.

In another example, voice commands are provided to define size of zoom region. In this example, the scaling factor and the size of the zoomed region and bounding box can be defined by the user through a voice command (e.g. zoom 3 times, zoom to a 2-by-2-by-2mm region of interest, bounding box size 4-by-4-by-5mm, etc.)

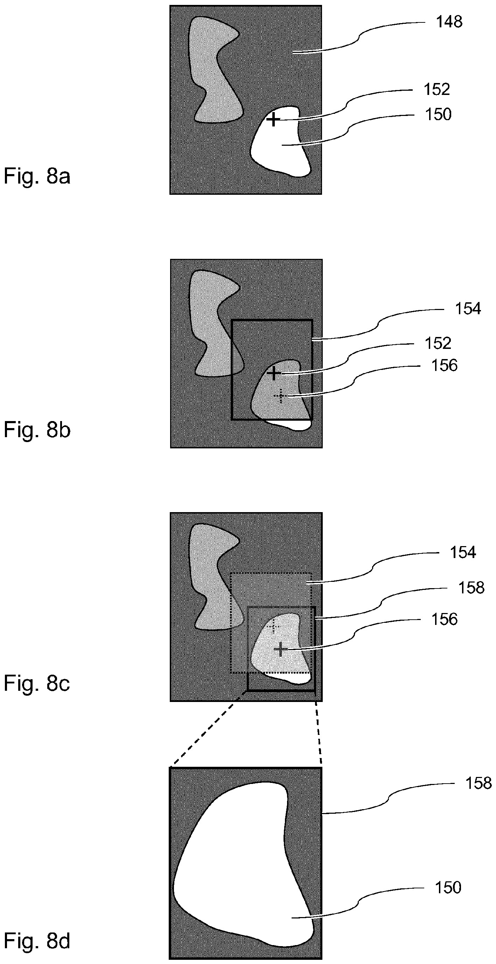

Fig. 8a to Fig. 8d show an example of a content-adaptive zoom-in and centering scheme. Fig. 8a shows an initial state with an anatomical structure 148 presented as 3D image with a portion of interest 150. A center mark 152 is arranged on the portion of interest 150. Fig. 8b indicates an identified (spatial) zoom field 154. The portion of interest 150 of the anatomical structure 148 is partly outside the zoom field 154. Thus, a recentering is provided to identify a new, i.e. adapted center mark 156 to define an adapted spatial zoom field 158. Fig. 8c shows a final state with cropped-off structures outside the adapted zoom field 158.

In another example, point & content-adaptive zoom is provided. As described above, Fig. 8a to Fig. 8d illustrate the steps involved in a content-adaptive zooming and centering scheme. Within the initial bounding box around the user-selected target location, an analysis of the image is performed to calculate a new optimal target center and target magnification factor. The zooming controller refines the target point provided by the user according to the visible content. A possible algorithm comprises calculating the center of mass of the voxels or mesh vertices contained within the initial bounding box and use that center of mass as the target center for the zoom operation. The mass of each point can be defined as the corresponding voxel value in a volumetric dataset, or the voxel opacity defined by the rendering transfer function. All invisible voxels are assigned a mass of 0 and visible voxels are assigned a mass value proportional to their opacity. For a mesh, the mass can be defined as 1 inside the object, 0 outside. The non-zero mass points that contribute to the center of mass calculations are shown as an overlay in Fig. 8b and Fig. 8c, and may include parts of several disjoint objects. The center of mass is shown as a red cross and will be the center of the new region of interest. The total volume of the region defined by the non-zero mass points can be used to estimate an optimal scaling factor, which can be chosen so that the final image contains a predefined ratio of opaque foreground voxels, e.g. 75%.

Fig. 9 shows another example of a presentation of an anatomical structure 168. A marker 170 is placed on a presented volume in a region of interest identifying a portion of interest for further zooming-in and cropping-off steps (not further shown in Fig. 9).

In another example, centering to labelled regions is provided. If 3D markers have been automatically or manually added to label regions of interest, they can be used as target centers as an alternative to pointing with fingers or eye gaze. Another embodiment of the zooming application utilizes the markers as zoom center points and using a voice command such as 'zoom + marker one', the model can zoom and center around the marker (see also Fig. 9). Markers can also be used as magnetic points for gaze and gesture pointing locations which allows the user to accurately zoom in around the marked anatomy without needing precise gesture pointing or eye gaze.

In an example of the device 10, the output interface is configured to present the first view to the user in a 3D mixed reality presentation. The data input 12 is configured to receive user interaction from at least one of the group of: manually pointing to a location within the 3D mixed reality presentation and pointing tracking, eye gazing for identifying the location within the 3D mixed reality presentation and voice commands.

The manual pointing relates to tracking fingertips, fingers or hands, or provided pointing devices.

The eye gazing relates to tracking viewing direction and focal distance, and also eye movement of eye blinking.

The voice commands relate to detecting and interpreting of words spoken by the user.

In an example of the device 10, the output interface 16 is configured to present the 3D mixed reality presentation as a presentation of the 3D image data as an overlay to reality such that the user can observe his/her hands within the presentation of the 3D image data. The data input 12 is configured to receive 3D hand tracking data from a measurement of a spatial position of the user's hand in relation to the presentation of the 3D image data.

The hand tracking can be provided by a tracking device. In an example, the tracking is provided based on camera images, e.g. from two or more cameras. In an example, the tracking is provided based on electromagnetic tracking of markers attached to the hand, e.g. by a glove.

In an example of the device 10, the data input 12 is configured to receive user adaptation of at least one of the parameters of the pre-determined zoom factor and the pre-determined bounding box. The data processor 14 is configured to generate the zoomed second view based on the at least one adapted parameter.

In an example of the device 10, the output interface 16 is configured to provide the first view comprising segmented anatomical structures. The output interface 16 is configured to provide the first view as 3D presentation to the user. The data input 12 is configured to receive the user interaction as spatial related user interaction provided within the 3D presentation. The user interaction is thus enabled within the 3D presentation of the 3D structure of the segmented anatomical structures.

In an example of the device 10, the data input 12 is configured to provide anatomical image content data for a predefined region around the determined location. The data processor 14 is configured to identify anatomical structures based on the determined location and the anatomical image content data. The data processor 14 is configured to adjust, based on the identified anatomical structures, at least one of the group of: location, bounding box and zoom center point to generate at least one of the group of an adjusted zoom center point and an adjusted bounding box such that a defined part of the anatomical structures is less cropped in the zoomed second view.

In addition, the content- and/or context-based scaling allows to automatically adapt to image content using image analysis algorithms that can identify the most important areas and select the optimal center and magnification factor accordingly.

In an example of the device 10, the data processor is configured to identify an anatomical portion. The data processor 14 is configured to at least one of the group of the following: i) adjust the zoom center point to a center of the anatomical portion; and ii) adjust the bounding box and/or the zoom factor to enclose a maximum of the anatomical portion.

The image content-based adjustment is provided in order to present the anatomical structure in a less cluttered way, i.e. in an optimized view.

In an example, for activating the zooming-in and the cropping, a trigger event is provided that comprises at least one of the group of: voice command, eye gaze, gesture recognition and manual interface interaction.

In an example, beside a 3D pointer for the user interaction, only a voice command interface is provided.

As trigger event, a voice command or gesture or other user interaction can be used. For the 3D pointing device, several options are provided comprising eye gaze, fingertip, middle of two fingertips and the like. The zooming controller is provided in several options.

In an example, a predefined constant magnification factor is applied, e.g. 1.5x or 2x, and the center is chosen to be the 3D target identified by the user with the pointing device. If two fingers are used, the magnification factor is determined by the distance between the two fingers, so that the content between those fingers is scaled to fit the whole bounding box. In addition, as an option, the volumetric content is analyzed in the neighborhood of the target point to derive an optimal content-adaptive scale factor and center.

In another example, other trigger events are provided. In this example, the magnification may be initiated by trigger events other than voice commands (e.g. maintaining the eye gaze or pointer for more than a specific time in one location while being in zoom mode, etc.).

In another example, adapting the bounding box to the content is provided. In this example, if some important structures on the sides of the bounding box were cropped after zoom and yet all other information inside the bounding box is necessary, the size of the bounding box is adjusted, e.g. manually, to include the cropped information without changing the scale or losing other important information. This may be done by selecting the individual sides or corners of the bounding box and dragging them towards outside. The reverse of this operation may also be provided to crop more information from the bounding box by reducing its size while maintaining the scale.

In an example of the device 10 (not shown in detail), the data processor 14 comprises a convolutional neural network configured to perform, based on the 3D image data, at least one of the following: annotate the 3D image data, determine a zoom factor, identify a procedure type, zoom-in and crop-off of content outside the bounding box.

In another example, convolutional neural networks are provided to refine the target point. This example describes a system that uses convolutional neural networks to identify the area of interest to be scaled and the proper scaling factor using anatomical landmarks and other procedure related information. This information includes the area or organ where the procedure is performed on, e.g. brain, spine, heart, kidney etc., pre-operative notes or diagnoses, e.g. aneurysm, thrombus, occlusion, stone etc., current device locations, e.g. catheter tip, stent, coil, etc., common practices and workflows, e.g. measuring the parent vessel diameter before aneurysm treatment, and the like.

The system describing this embodiment consists of an interventional imaging system and a training phase neural network controller. The interventional imaging system is provided to generate the 3D images. The image processing controller detects and annotates the target volumetric region of interest in 3D images. The annotation may be indicated using bounding box, centroid, binary segmentation etc. The annotations may be generated using any of the methods, traditional computer vision-based or deep learning based, established in the art (e.g. thresholding, region growing, template matching, level sets, active contour modelling, neural networks (e.g., U-nets), manual or semi-automatic method, etc.. The training phase neural network controller is configured to receive retrospective data from a relatively large (N >> 100) patient population (covering various anatomies, abnormalities, age groups, genders, BMI, etc.). The data comprises 3D images (at least one per patient) containing at least one of the types of the abnormalities (e.g. aneurysm, bone or spine injury, heart valve issue, kidney stone, etc.). The location of the target may be manually annotated by expert users or automatically annotated as enumerated above. The scaling factor may be manually selected by users for best view of the target volumetric region.

The training phase neural network controller is also configured to train a neural network (for instance, a convolutional neural network) using at least one target location and scaling factor on each 3D volume. Multiple targets in this network may come from the same or various stages in the procedure, such as multiple aneurysms in the 3D image or images from different viewing angles, etc.

The network may include several fully connected or convolutional layers with pooling, normalization, non-linear layers, etc. between them. The network produces a n x 1 vector output where n is the number of values regressed by the network (e.g. XYZ location of the target, scaling factor, XYZ locations of eight corners of the bounding box relative to the original volume, etc.). Errors are computed by comparing these values produced by the neural network with ground truth values using some loss function (e.g. L1 or L2 loss, Huber loss, logcosh loss, etc.) and used to perform stochastic gradient descent to optimize network weights.

At inference time, input the 3D images as well as any information that could help identifying the procedure type or site to the trained neural network is provided. The network will then zoom into the region of interest.

The trained network may also assign confidence estimates to the predictions, for instance, by observing the attention maps produced by the network and evaluating whether the attention of the network was within the region of interest, e.g. at or around a target aneurysm.

In an example of the device 10, the data input 12 is configured to provide markers as labels of regions of interest of the 3D image data, wherein the markers are provided as identifiable zoom center points for the second view.

The term "subject" may also be referred to as individual. The "subject" may further also be referred to as patient, although it is noted that this term does not indicate whether any illness or disease is actually present with the subject.

In another exemplary embodiment of the present invention, a computer program or a computer program element is provided that is characterized by being adapted to execute the method steps of the method according to one of the preceding embodiments, on an appropriate system.

In an example, a computer program or program element for controlling an apparatus according to one of the examples above is provided, which program or program element, when being executed by a processing unit, is adapted to perform the method steps of one of the method examples above.

The computer program element might therefore be stored on a computer unit or be distributed over more than one computer units, which might also be part of an embodiment of the present invention. This computing unit may be adapted to perform or induce a performing of the steps of the method described above. Moreover, it may be adapted to operate the components of the above-described apparatus. The computing unit can be adapted to operate automatically and/or to execute the orders of a user. A computer program may be loaded into a working memory of a data processor. The data processor may thus be equipped to carry out the method of the invention.

Aspects of the invention may be implemented in a computer program product, which may be a collection of computer program instructions stored on a computer readable storage device which may be executed by a computer. The instructions of the present invention may be in any interpretable or executable code mechanism, including but not limited to scripts, interpretable programs, dynamic link libraries (DLLs) or Java classes. The instructions can be provided as complete executable programs, partial executable programs, as modifications to existing programs (e.g. updates) or extensions for existing programs (e.g. plugins). Moreover, parts of the processing of the present invention may be distributed over multiple computers or processors.

As discussed above, the processing unit, for instance a controller implements the control method. The controller can be implemented in numerous ways, with software and/or hardware, to perform the various functions required. A processor is one example of a controller which employs one or more microprocessors that may be programmed using software (e.g., microcode) to perform the required functions. A controller may however be implemented with or without employing a processor, and also may be implemented as a combination of dedicated hardware to perform some functions and a processor (e.g., one or more programmed microprocessors and associated circuitry) to perform other functions.

Examples of controller components that may be employed in various embodiments of the present disclosure include, but are not limited to, conventional microprocessors, application specific integrated circuits (ASICs), and field-programmable gate arrays (FPGAs).

This exemplary embodiment of the invention covers both, a computer program that right from the beginning uses the invention and a computer program that by means of an update turns an existing program into a program that uses the invention.

Further on, the computer program element might be able to provide all necessary steps to fulfil the procedure of an exemplary embodiment of the method as described above.

According to a further exemplary embodiment of the present invention, a computer readable medium, such as a CD-ROM, is presented wherein the computer readable medium has a computer program element stored on it, which computer program element is described by the preceding section. A computer program may be stored and/or distributed on a suitable medium, such as an optical storage medium or a solid-state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated, and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfil the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. A device (10) for guidance in medical interventions, comprising:
- a data input (12);
- a data processor (14); and
- an output interface (16);
wherein the data input is configured: to provide 3D image data of a region of interest of a subject; to provide a zoom factor and a bounding box around the determined location; and to receive a user interaction for defining locations within the region of interest in the 3D image data;
wherein the data processor is configured: to determine, by the user interaction with a presented first view, a location within the presentation of the region of interest as zoom center point for a zoomed second view; to zoom-in of the 3D image data according to the zoom factor; to crop-off content of the 3D image data outside the bounding box defining a new region of interest inside the bounding box; and to generate the zoomed second view for the new region of interest based on the zoom factor and the 3D image data within the bounding box; and
wherein the output interface is configured: to provide, based on the 3D image data, presentation data for the region of interest in the first view; and to provide presentation data for the new region of interest in the zoomed second view.

2. Device according to claim 1, wherein the output interface is configured to present the first view to the user in a 3D mixed reality presentation; and
wherein the data input is configured to receive user interaction from at least one of the group of:
- manually pointing to a location within the 3D mixed reality presentation and pointing tracking;
- eye gazing for identifying the location within the 3D mixed reality presentation; and
- voice commands.

3. Device according to claim 2, wherein the output interface is configured to present the 3D mixed reality presentation as a presentation of the 3D image data as an overlay to reality such that the user can observe his/her hands within the presentation of the 3D image data; and
wherein the data input is configured to receive 3D hand tracking data from a measurement of a spatial position of the user's hand in relation to the presentation of the 3D image data.

4. Device according to one of the claims 1 to 3, wherein the data input is configured to receive user adaptation of at least one of the parameters of the pre-determined zoom factor and the pre-determined bounding box; and
wherein the data processor is configured to generate the zoomed second view based on the at least one adapted parameter.

5. Device according to one of the preceding claims, wherein the output interface is configured to provide the first view comprising segmented anatomical structures;
wherein the output interface is configured: to provide the first view as 3D presentation to the user; and
wherein the data input is configured: to receive the user interaction as spatial related user interaction provided within the 3D presentation.

6. Device according to one of the preceding claims, wherein the data input is configured to provide anatomical image content data for a predefined region around the determined location;
wherein the data processor is configured to identify anatomical structures based on the determined location and the anatomical image content data; and
wherein the data processor is configured to adjust, based on the identified anatomical structures, at least one of the group of: location, bounding box and zoom center point to generate at least one of the group of an adjusted zoom center point and an adjusted bounding box such that a defined part of the anatomical structures is less cropped in the second view.

7. Device according to one of the preceding claims, wherein the data processor is configured to identify an anatomical portion; and
wherein the data processor is configured to at least one of the group of the following:
i) adjust the zoom center point to a center of the anatomical portion; and
ii) adjust the bounding box and/or the zoom factor to enclose a maximum of the anatomical portion.

8. Device according to one of the preceding claims, wherein the data processor comprises a convolutional neural network configured to perform, based on the 3D image data, at least one of the following:
- annotate the 3D image data;
- determine a zoom factor;
- identify a procedure type;
- zoom-in; and
- crop-off of content outside the bounding box.

9. Device according to one of the preceding claims, wherein the data input is configured to provide markers as labels of regions of interest of the 3D image data, wherein the markers are provided as identifiable zoom center points for the second view.

10. A system (50) for guidance in medical interventions, comprising:
- a display arrangement (52);
- a user input device (54); and
- a device (10) for guidance in medical interventions according to one of the preceding claims;
wherein the display arrangement is data-connected to the device for guidance in medical interventions and is configured to provide a 3D presentation of a region of interest of a subject; and
wherein the user input device is data-connected to the device for guidance in medical interventions and is configured for user interaction to determine a location within the 3D presentation.

11. System according to claim 10, wherein the display arrangement comprises a stereo display (58) configured to present the 3D image data for spatial user action within the presented 3D image.

12. System according to claim 10 or 11, wherein the display arrangement is a mixed reality display (60) configured to present the 3D image data overlaid to reality for user interaction by 3D pointing at a location within the 3D image; and
wherein the user input device comprises a detector for detecting the pointing of the user.

13. A method (200) for guidance in medical interventions, comprising the following steps:
- providing (202) 3D image data of a region of interest of a subject;
- presenting (204), based on the 3D image data, the region of interest in a first view;
- determining (206), by user interaction with the presented first view, a location within the presentation of the region of interest as zoom center point for a zoomed second view;
- providing (208) a zoom factor and a bounding box around the determined location;
- zooming-in (210) of the 3D image data according to the zoom factor;
- cropping-off (212) content of the 3D image data outside the bounding box defining a new region of interest inside the bounding box;
- generating (214) the zoomed second view for the new region of interest based on the zoom factor and the 3D image data within the bounding box; and
- providing (216) the zoomed second view to the user.

14. A computer program comprising instructions which, when the program is executed by a computer, cause the computer to carry out the method of claim 13.

15. A computer readable medium having stored the computer program of claim 14.
